# EUROPEAN PATENT APPLICATION

(11) **EP 3 611 181 A1**
(43) Date of publication of application: **19.02.2020**
(21) Application number: 18761386.4
(22) Date of filing: 02.02.2018
(51) Int. Cl.: C07K 7/06, C07K 7/56, A61K 47/64, A61K 51/08, A61K 49/00, A61P 35/00, A61K 101/02, A61K 101/00

(54) **ATP1A1-TARGETING POLYPEPTIDE AND USE**

(30) Priority: 01.03.2017 CN 201710117296
(71) Applicant: Sun Yat-Sen University Cancer Center (SYSUCC), Guangzhou Guangdong 510060 (CN)
(72) Inventor: ZENG, Musheng, Guangzhou Guangdong 510060 (CN); FENG, Guokai, Guangzhou Guangdong 510060 (CN); WANG, Qian, Guangzhou Guangdong 510060 (CN); YE, Xiaoxuan, Guangzhou Guangdong 510060 (CN); XIAO, Wei, Guangzhou Guangdong 510060 (CN); LI, Manzhi, Guangzhou Guangdong 510060 (CN)
(74) Representative: Gassner, Birgitta
(86) International application number: PCT/CN2018/075056
(87) International publication number: WO 2018/157696

(57) **Abstract**

The invention discloses an ATP1A1-targeting polypeptide and the use thereof. The motif of the ATP1A1-targeting polypeptide is represented by the general formula of SISSLTH, and the C- and/or N-terminal(s) of the motif is/are optionally linked with 1-3 amino acids. The ATP1A1-targeting polypeptide can target ATP1A1 well, and has great application value in the molecular diagnosis and targeted therapy of tumors.

SEQUENCE LISTING

<110>  SUN YAT-SEN UNIVERSITY CANCER CENTER (SYSUCC)

<120>  ATP1A1-targeting poplypeptide and use thereof

<130>

<160>  4

<170>  PatentIn version 3.5

<210>  1
<211>  9
<212>  PRT
<213>  Artificial polypeptide

<400>  1

Cys Ser Ile Ser Ser Leu Thr His Cys
1               5

<210>  2
<211>  9
<212>  PRT
<213>  Artificial polypeptide

<400>  2

Cys Gly Gly Gly Gly Gly Gly Gly Cys
1               5

<210>  3
<211>  13
<212>  PRT
<213>  Artificial polypeptide

<400>  3

Cys Ser Ile Ser Ser Leu Thr His Cys Gly Gly Gly Ser
1               5                   10

<210>  4
<211>  13
<212>  PRT
<213>  Artificial polypeptide

<400>  4

Cys Gly Gly Gly Gly Gly Gly Gly Cys Gly Gly Gly Ser
1               5                   10

## Description

### FIELD OF THE INVENTION

This invention relates to a targeting polypeptide, and more particularly, to an ATP1A1-targeting polypeptide and the use thereof.

### BACKGROUND OF THE INVENTION

Tumor-targeting preparations are generally polypeptide-based, which are typically enriched in tumor blood vessels or tumor cells by specifically binding to the molecules on tumor cell surfaces or tumor blood vessels.

Tumor-targeting polypeptides have great value in the application for the molecular diagnosis and targeted therapy of tumors. On one hand, tumor-targeting polypeptides may be applied to tumor molecular imaging through coupling with molecular imaging reagents. On the other hand, tumor-targeting polypeptides may be applied to tumor targeted therapy by coupling with anti-tumor drugs. For example, Prof. Ruoslahti Errki, a member of the US Academy of Sciences, discovered a class of tumor-targeting polypeptides containing RGD motifs. This class of tumor-targeting polypeptides binds to the integrin av in tumor neovascularization. According to the research results of Prof. Ruoslahti Errki, many tumor-targeting polypeptides containing RGD motifs have entered various stages of clinical trials, and some of them have shown good effects.

By coupling with various molecular imaging reagents, tumor-targeting polypeptides can be applied to optical imaging, PET, SPECT and MRI. Tumor molecular imaging is significant for locating tumor cells at molecular levels, for early diagnosis of tumors, and for tracking residual lesions and metastases. At present, many polypeptides targeting various tumor markers, such as HER2, EphA2, and MMP-9, have been used in the imaging of tumor models.

Imageological examinations are effective methods for tumor diagnosis. However, existing imageological examinations can only distinguish normal tissues from tumor tissues at a tissue-resolution level, and can hardly distinguish malignant tumors from benign hyperplasia accurately at a molecular level.

ATP1A1 is highly expressed in esophageal cancer, breast cancer, melanoma and liver cancer. By now, there is no report about the use of ATP1A1 as a target for polypeptide molecular imaging of tumors. An ATP1A1-targeting polypeptide can not only be used to detect the expression of ATP1A1 in tumor patients to indicate the staging of tumors and evaluate the therapeutic effect of ATP1A1-targeting therapeutic drugs, but also be used to track micrometastases of tumors. Thus, it is of great practical importance to develop an ATP1A1-targeting peptide.

### SUMMARY OF THE INVENTION

The object of the invention is to provide an ATP1A1-targeting polypeptide and the use thereof. The technical solutions of the invention are as follows.

In one aspect of the invention, an ATP1A1-targeting polypeptide is provided. The ATP1A1-targeting polypeptide has a motif of SISSLTH with the C- and/or N-terminal(s) of the motif optionally linked with 1-3 amino acids.

As a further improvement of the ATP1A1-targeting polypeptide as defined above, the polypeptide may form into a ring through amino acids at the terminals thereof.

As a further improvement of the ATP1A1-targeting polypeptide as defined above, each of the two terminals of motif of the polypeptide may be linked with one Cys. Further, the polypeptide may form into a ring through Cys at both terminals of the motif.

In another aspect of the invention, a tumor-targeting therapeutic preparation is provided. The tumor-targeting therapeutic preparation comprises an active molecule having therapeutic effect on a tumor, and the active molecule is coupled with the ATP1A1-targeting polypeptide as defined above.

As a further improvement of the tumor-targeting therapeutic preparation as defined above, the active molecule may be a chemotherapeutic drug of a tumor.

In yet another aspect of the invention, a diagnostic reagent for tumor is provided. The diagnostic reagent for tumor comprises an imaging group, and the imaging group is coupled with the ATP1A1-targeting polypeptide as defined above.

As a further improvement of the diagnostic reagent for tumor as defined above, the imaging group may be a radionuclide, a fluorophore. In particular, the imaging group may be selected from ^{99m}Te, ¹⁸F, or a fluorophore Cy5.

The invention may have the following beneficial effects.

The polypeptide of the invention has excellent targeting properties and can bind to ATP1A1 with high affinity.

The polypeptide of the invention, when coupled with a molecular marker, can bind tumor cells well, and thus can be advantageously applied to tumor screening and diagnosis.

In addition, the polypeptide of the invention, when coupled with a drug molecule, may have a targeted therapeutic effect.

### BRIEF DESCRIPTION OF THE DRAWINGS

- Figure 1: shows the results of tumor-targeting experiments.
- Figure 2: shows infrared fluorescent molecular imaging of tumor-bearing mice.
- Figure 3: shows SPECT molecular imaging of tumor-bearing mice.

### DETAILED DESCRIPTION OF THE EMBODIMENTS

The invention provides a tumor-targeting preparation, which is coupled with any one of the ATP1A1-targeting polypeptides as defined above. In such a preparation, the polypeptide plays a role of targeting, and the effector molecule coupled thereto may be a diagnostic reagent, or a therapeutic drug such as a tumor-killing peptide, a small molecular drug for chemotherapy, or the like. Those commonly used coupling groups in the art can be used to ensure the well coupling of the effector molecule with the polypeptide. In addition, a non-binding site outside or on the motif shall be selected as the coupling site, in order to ensure the targeting effect.

The ATP1A1-targeting polypeptide as defined above can be composed of L-amino acids or D-amino acids.

The peptides used in the following experiments are listed as follows.

| Name of Peptide | Sequence (cyclization with the two italic *C*) | SEQ ID NO: |
|---|---|---|
| S3 | *C*SISSLTH*C* | 1 |
| Con | *C*GGGGGGG*C* | 2 |
| Biotin-S3 | Biotin-*C*SISSLTH*C* | |
| Biotin-C | Biotin-*C*GGGGGGG*C* | |
| Cy5-S3 | Cy5-*C*SISSLTH*C* | |
| Cy5-C | Cy5-*C*GGGGGGG*C* | |
| FITC-S3 | *C*SISSLTH*C*-*GGGS*-FITC | 3 |
| FITC-C | *C*GGGGGGG*C*-*GGGS*-FITC | 4 |
| HYNIC-S3 | HYNIC-*C*SISSLTH*C* | |
| HYNIC-C | HYNIC-*C*GGGGGGG*C* | |

### Data analysis

Counting results were analyzed by the statistical software SPSS16.0, and the variance homogeneity test was conducted for the counting results of various groups by Levene's test. When the variances of the groups were homogeneous (if P>0.05, the variance was homogeneous), an independent sample t test was used to analyze and determine whether there was a significant difference between the groups (if P<0.05, there was a significant difference).

### Tumor-targeting experiment

An enzyme-linked immunosorbent assay was used to detect the binding of S3 phage to breast cancer MDA-MB-231 cells, and then to detect whether S3 peptide could block the binding of S3 peptide to breast cancer MDA-MB-231 cells. The specific procedure was as follows:
1) counting the titer of phage, and setting aside for use;
2) digesting cells with trypsin, counting, and uniformly plating the cells in a 96-well plate, 1 × 10⁴/ well;
3) in the blocking experiment, adding 5, 10, 20 µg/µl S3 polypeptide and incubating at 37°C for 1 h;
4) in the targeting experiment, adding phages in a concentration gradient of 1.0×10⁹, 2.0×10⁹, 4.0×10⁹, 8.0×10⁹, 1.6×10⁹, 3.2×10⁹ pfu/well, incubating at 37°C for 2 h for binding; in the blocking experiment, adding 20 µg/µl of Biotin-S3 polypeptide;
5) discarding the cell incubation solution;
6) fixing the cells with 4% paraformaldehyde for 10 min;
7) washing five times with 0.1% PBST, 270 µl/well;
8) destroying the membranes with 0.1% Triton for 5 min, and washing once with PBST, 270 µl/well;
9) blocking with 5% BSA for 30 min;
10) adding 1:1000 diluted anti-M13conjucted HRP by 50 µl/well, and incubating at room temperature for 1 h;
11) washing five times with 0.1% PBST;
12) developing color with a TMB kit; and
13) reading at OD 450nm by a microplate reader.

The binding of FITC-S3 fluorescent peptide to breast cancer MDA-MB-231 cells was detected by flow cytometry. The inventors first synthesized a fluorescent peptide in which the 35 peptide and Con peptide were conjugated to a fluorescent dye FITC, and then incubated FITC-S3 fluorescent peptide with the cells, and finally detected the binding thereof by flow cytometry. The particular experimental procedure was as follows:
1) digesting cells with trypsin, counting, and adjusting the concentration to be 1.0×10⁶ cells/tube, resuspending the cells with 100µl serum-free 1640 medium;
2) adding FITC-C or FITC-35 in PBS (10 µM), and incubating the cells on ice for binding for 20 min;
3) washing three times with 0.1% PBST, 1 ml/time, and centrifuging at 400 g for 5 min;
4) resuspending the cells with 500 µl PBS; and
5) detecting by flow cytometry.

The binding of Biotin-C and Biotin-S3 polypeptide to human breast cancer MDA-MB-231 cells was detected by confocal laser scanning microscopy. Meanwhile, the inventors compared it with the binding of the above polypeptides to human normal breast MCF-10A cells. The particular experimental procedure was as follows:
1) digesting cells with trypsin and plating the cells in a 24-well plate provided with a slide, 1×10⁵ cells/well;
2) adding Biotin-C, Biotin-S3 polypeptide to the cells, 200µg/well, and incubating at 37°C for 2h;
3) discarding the cell incubation solution;
4) fixing the cells with 4% paraformaldehyde for 10 min;
5) washing five times with 0.1% PBST, 500 µl/well, and washing off unbound polypeptides;
6) destroying the membranes with 0.1% Triton for 5 min, and washing once with PBST, 500 µl/well;
7) blocking with 5% BSA, 30 min;
8) adding anti-Biotin Streptavidin (1:1000) into the wells, and incubating at 4°C for 12 h;
9) washing three times with 0.1% PBST, 5 min/time;
10) adding anti-Streptavidin conjugated FITC (1:1000) into the wells, and incubating at room temperature for 45 min;
11) washing three times with 0.1% PBST, 5 min/time;
12) staining cell membranes with WGA (1:1000), and incubating at room temperature for 10 min;
13) washing three times with 0.1% PBST, 5 min/time;
14) staining cell nuclei with DAPI (1:3000), and incubating at room temperature for 5 min;
15) washing three times with 0.1% PBST, 5 min/time; and
16) mounting with anti-fade.

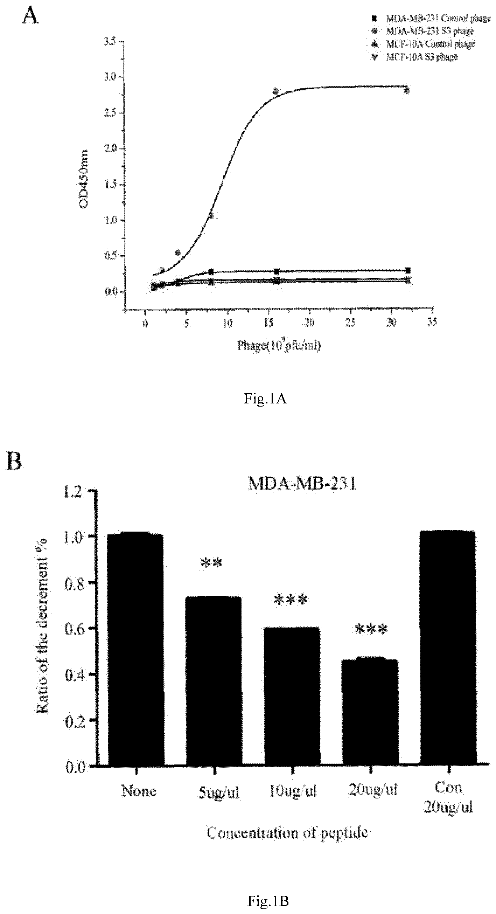

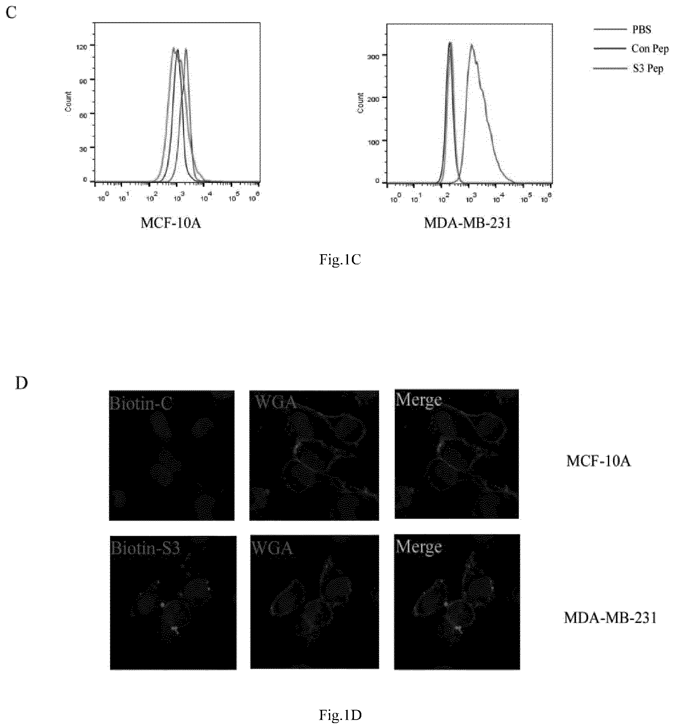

The experimental results were shown in Fig. 1. The phage binding experiment showed that, at 37 °C, the binding ability of S3 phages to breast cancer MDA-MB-231 cells was stronger than that of the control phage, and increased as the phage titer increased (Fig. 1 A). The polypeptide blocking experiment showed that S3 polypeptide could block the binding of Biotin-S3 polypeptide to breast cancer MDA-MB-21 cells, and the blocking effect was concentration-dependent (Fig. 1B). The results of flow cytometry showed that the binding ability of FITC-S3 fluorescent peptide to breast cancer MDA-MB-231 cells was stronger than that of FITC-control fluorescent peptide (Fig. 1C). The immunofluorescence showed that Biotin-S3 polypeptide could bind to the surfaces of the cell membranes (Fig. ID). In view of the above, S3 peptide has an obvious property of targeting breast cancer cells as compared to normal breast MCF-10A cells.

### Infrared fluorescent molecular imaging of tumors

Tumor-bearing mice were used for the infrared fluorescence molecular imaging experiment to detect the distribution of Cy5-S3 fluorescent peptide in various organs and tumors of the tumor-bearing mice. The experimental procedure of the imaging experiment of small living animals was as follows:
1) taking out nine male NOD SCID mice of 4-week old;
2) digesting breast cancer MDA-MB-231 cells with trypsin, counting, and adjusting the concentration to be 5.0 × 10⁷ cells/ml, resuspending the cells with 20% Matrigel DMEM, and injecting with 200 µl/mouse;
3) observing tumor formation for 1-2 weeks, measuring the sizes of the tumors, and randomizing the mice into three groups according to the sizes of the tumors, i.e. PBS group, control peptide Cy5-C group and Cy5-S3 group, with 3 mice in each group;
4) dissolving 300µg fluorescent peptides in 200µl PBS, injecting the fluorescent peptides into the tail veins of the mice, and dynamically detecting the distribution of the fluorescent peptides in various organs and tumors of the nude mice by using a small living animal imaging instrument; and
5) taking out every organ and tumor, homogenizing, measuring the fluorescence intensity of each organ and tumor, and making statistical analysis.

According to the above procedure, the Cy5-S3 fluorescent peptides were applied in esophageal cancer KYSE30 tumor-bearing and nasopharyngeal carcinoma CNE2 mouse models, in order to detect whether Cy5-S3 fluorescent peptide can be imaged with infrared fluorescent molecular imaging in other tumors.

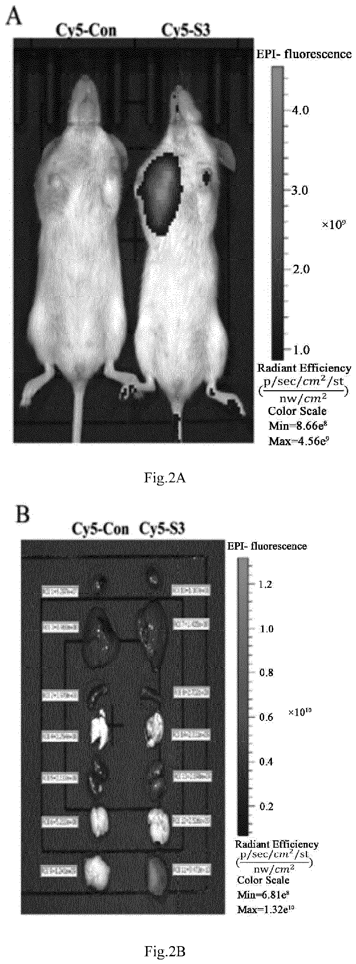

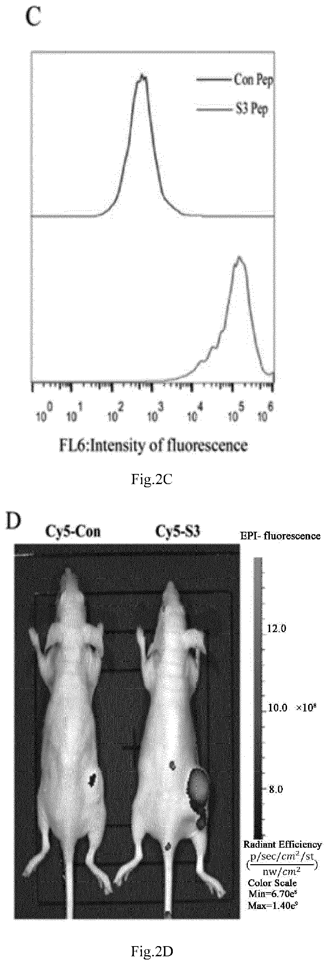

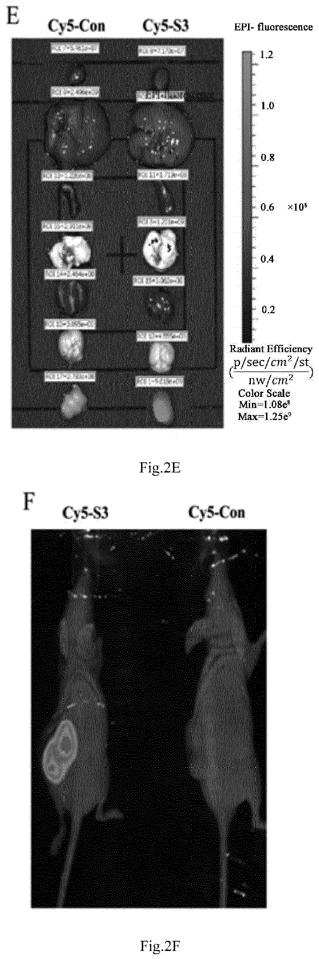

The experimental results were shown in Fig. 2. After 48 hours from tail vein injection, the mice were subjected to infrared imaging. It can be seen that the Cy5-S3 fluorescent peptides were obviously enriched in the tumors, and almost no fluorescent peptides were enriched in other organs (Fig. 2A). The mice were dissected for tissue analysis, which showed that the Cy5-S3 fluorescent peptides were significantly enriched in the tumor, but not enriched in other organs (Fig. 2B). After 48 hours from tail vein injection, the content of the Cy5-S3 fluorescent peptides in unit weight of the tumor was significantly higher than that in other organs (Fig. 2C). In addition, the Cy5-S3 fluorescent peptide can also be used for the infrared fluorescent molecular imaging of the esophageal cancer (Figs. 2D, E) and nasopharyngeal carcinoma (Fig. 2F) after 48 hours from tail vein injection. These results demonstrate that, the S3 peptide can be applied to the living body molecular imaging of various tumor models (e.g. breast cancer, esophageal cancer, and nasopharyngeal carcinoma).

### SPECT molecular imaging experiment of tumors

An HYNIC-modified 35-peptide labeled with ^{99m}Tc was applied to SPECT imaging of tumor-bearing mice. The experimental procedure was as follows:
1) dissolving 100µg HYNIC-35 polypeptide in 100µl EDDA/tricine solution (20 mg/ml tricine, 10 mg/ml EDDA, pH 7);
2) adding 20µl tin(II) solution (10 mg SnCl₂•2H₂O dissolving in 10 ml 0.1 N HCl);
3) adding 1ml ^{99m}TcO4 (800MBq) solution;
4) heating at 100°C for 10 min and then cooling to room temperature;
5) injecting into the tail veins of the tumor-bearing nude mice with 100µl/mice;
6) detecting the distribution of HYNIC-S3 peptide with SPECT at 0.5 h, 1 h and 2 h after the injection.

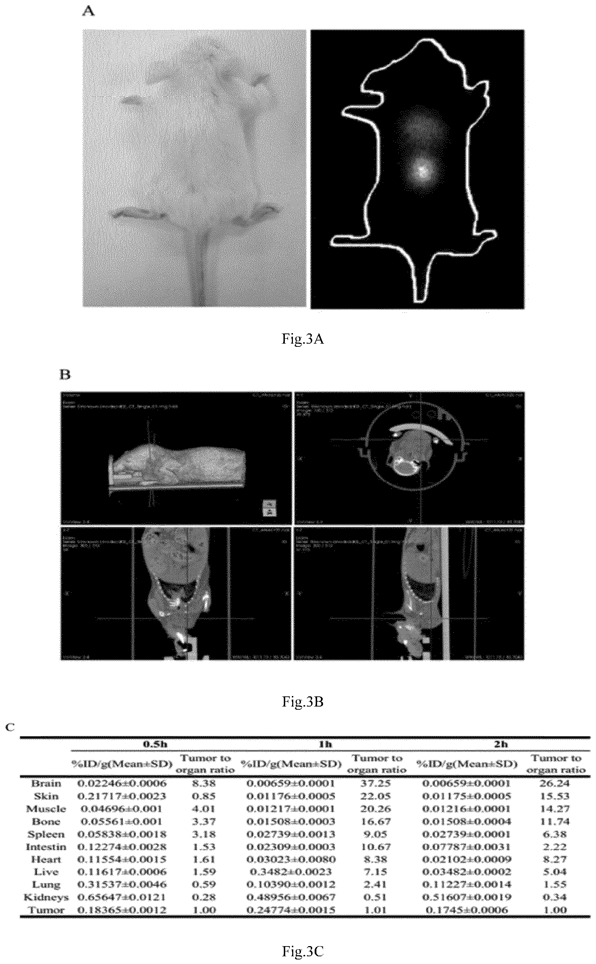

The SPECT experimental results showed that HYNIC-S3 peptides were mainly distributed in tumors and kidneys after 1 hour from injection (Figs. 3A and C). The tumor sites of the mice were imaged by small animal CT (Fig. 3B). These results demonstrate that the HYNIC-S3 peptide can be applied to SPECT imaging of tumors.

Obviously, the ARP1A1 targeting polypeptide of the invention can be excellently used for the diagnosis and targeted therapy of tumors, especially for the molecular imaging of tumors.

## Claims

1. An ATP1A1-targeting polypeptide, **characterized in that**, the ATP1A1-targeting polypeptide has a motif of SISSLTH with the C- and/or N-terminal(s) of the motif optionally linked with 1-3 amino acids.

2. The ATP1A1-targeting polypeptide according to claim 1, **characterized in that**, the polypeptide forms into a ring through amino acids at the terminals thereof.

3. The ATP1A1-targeting polypeptide according to claim 1 or 2, **characterized in that**, each of the two terminals of motif of the polypeptide is linked with one Cys.

4. Use of the ATP1A1-targeting polypeptide according to any one of claims 1-3 in the manufacture of a diagnostic reagent for tumor.

5. Use of the ATP1A1-targeting polypeptide according to any one of claims 1-3 in the manufacture of a tumor-targeting drug.

6. A tumor-targeting therapeutic preparation, comprising an active molecule which has a therapeutic effect on a tumor, **characterized in that**, the active molecule is coupled with the ATP1A1-targeting polypeptide according to any one of claims 1-3.

7. The tumor-targeting therapeutic preparation according to claim 6, **characterized in that**, the active molecule is a chemotherapeutic drug of a tumor.

8. A diagnostic reagent for tumor, comprising an imaging group, **characterized in that**, the imaging group is coupled with the ATP1A1-targeting polypeptide according to any one of claims 1-3.

9. The diagnostic reagent for tumor according to claim 8, **characterized in that**, the imaging group is a radionuclide, a fluorophore.

10. The diagnostic reagent for tumor according to claim 8, **characterized in that**, the imaging group is selected from ^{99m}Te, ¹⁸F, a fluorophore Cy5.
